# NEUE EUROPÄISCHE PATENTSCHRIFT

(11) **EP 0 784 609 B2**
(45) Veröffentlichungstag und Bekanntmachung des Hinweises auf die Entscheidung über den Einspruch: **19.01.2005**
(45) Hinweis auf die Patenterteilung: 28.07.1999
(21) Anmeldenummer: 95935377.2
(22) Anmeldetag: 25.09.1995
(51) Int. Cl.: C07C 233/36, C07C 231/12, C11D 1/94

(54) **VERFAHREN ZUR HERSTELLUNG VON PUMPFÄHIGEN WÄSSRIGEN TENSIDKONZENTRATEN**
PROCESS FOR MAKING PUMPABLE AQUEOUS TENSIDE CONCENTRATES
PROCEDE DE FABRICATION DE CONCENTRES DE TENSIOACTIFS AQUEUX POMPABLES

(30) Priorität: 04.10.1994 DE 4435495
(43) Veröffentlichungstag der Anmeldung: 23.07.1997
(73) Patentinhaber: Cognis Deutschland GmbH & Co. KG, 40589 Düsseldorf (DE)
(72) Erfinder: UPHUES, Günter, D-40789 Monheim (DE); KAHRE, Jörg, D-40789 Monheim (DE); BEHLER, Ansgar, D-46240 Bottrop (DE); NEUMANN, Peter, D-40589 Düsseldorf (DE); HENSEN, Hermann, D-42781 Haan (DE); SEIPEL, Werner, D-40723 Hilden (DE); TESMANN, Holger, D-41363 Jüchen (DE)
(86) Internationale Anmeldenummer: PCT/EP1995/003797
(87) Internationale Veröffentlichungsnummer: WO 1996/010558

(56) Entgegenhaltungen:
- EP-A- 0 353 580
- EP-A- 0 508 507
- EP-A- 0 510 870
- WO-A-91/14761
- WO-A-93/25650
- WO-A-95/04592
- WO-A-95/14658
- JP-A- 1 069 695
- JP-A- 2 187 499

## Beschreibung

Die Erfindung betrifft ein Verfahren zur Herstellung pumpfähiger wäßriger Tensidkonzentrate mit einem Gehalt an Alkyl- und/oder Alkenyloligoglykosiden und Betaintensiden.

### Stand der Technik

Alkyloligoglykoside, insbesondere Alkyloligoglucoside, stellen nichtionische Tenside dar, die infolge ihrer ausgezeichneten Detergenseigenschaften und hohen ökotoxikologischen Verträglichkeit zunehmend an Bedeutung gewinnen. Herstellung und Verwendung dieser Stoffe sind gerade in letzter Zeit in einer Reihe von Übersichtsartikeln dargestellt worden, von denen stellvertretend die Veröffentlichungen von H.Hensen in **Skin Care Forum, 1**, **(Okt. 1992),** D.Balzer und N.Ripke in **Seifen-Öle-Fette-Wachse 118, 894 (1992)** und B.Brancq in **Seifen-Öle-Fette-Wachse 118, 905 (1992)** genannt werden sollen.

In mancher Hinsicht ist der Einsatz von Alkyloligoglucosiden jedoch mit Problemen behaftet. So lassen sich beispielsweise keine pumpfähigen wäßrigen Konzentrate mit einem Feststoffgehalt oberhalb von 40 Gew.-% herstellen, ohne daß es im Zuge der Aufkonzentrierung zu einer partiellen Zersetzung der Zukkerkomponente kommt. Diese Eigenschaft haben die Glykoside übrigens mit den meisten anionischen Tensiden gemeinsam, die oberhalb eines Aktivsubstanzgehaltes von etwa 35 Gew.-% eine zähflüssige Gelphase bilden. Alkyloligoglucoside zeigen ferner die Tendenz, im Laufe einer Lagerung bei niedrigen Temperaturen zu kristallisieren, was ihre weitere Verwendung signifikant erschwert.

Die gemeinsame Verwendung von Alkylglucosiden zusammen mit amphoteren bzw. zwitterionischen Tensiden vom Betaintyp in oberflächenaktiven Mitteln ist aus dem Stand der Technik grundsätzlich bekannt.

Erstmalig offenbart werden Mischungen von - allerdings kurzkettigen - Alkylglucosiden und Alkylamidobetainen bzw. Imidazoliniumbetainen in einem Aufsatz von G.Proserpio et al. in **Rivista Italiana 56**, **567 (1974)**. In der **EP-A 0075994** (Procter & Gamble) werden Kombinationen von Alkylglucosiden mit Aminoxiden, ungesättigten Seifen, wasserlöslichen Buildern und ausgewählten Aniontensiden offenbart. Die Mischungen können ferner amphotere Tenside, beispielsweise Betaine vom Typ des 6-(-N-dodecylbenzyl-N,N-dimethylammonium)hexanoats enthalten. Aus der **US 4668422** (Henkel Corp.) sind Flüssigseifen und Schaumbäder mit einem Gehalt an Alkylglucosiden, Betainen und Aminoxiden bekannt. Gegenstand der **EP-A 0250181** (Helene Curtis) sind Flüssigwaschmittel, enthaltend Alkylglucoside, anionische Tenside und ausgewählte amphotere Tenside mit Betainstruktur. In der **EP-A 0341071** (Unilever) werden Tensidkombinationen offenbart, enthaltend Alkylglucoside, Alkylsulfate, Betaine und/oder Aminoxide und gegebenenfalls Alkanolamide. Manuelle Geschirrspülmittel, enthaltend Alkylglucoside, Fettalkoholsulfate, Fettalkoholethersulfate und Betaine sind aus den Schriften **EP-A 0513138, DE-A1 4234487** und **DE-A1 4311114** (alle Henkel) bekannt. In der **EP-A 0453238** (Unilever) werden milde Shampoos auf Basis von Alkylglucosiden, anionischen Tensiden und Betainen beschrieben. Die **EP-A 0508507** (Berol Nobel) betrifft schließlich Flüssigwaschmittel mit einem Gehalt an Alkylglucosiden, anionischen Tensiden und ausgewählten amphoteren Tensiden mit Betainstruktur. Alle diese Druckschriften haben jedoch verdünnte wäßrige Tensidgemische oder Mittel und keine Konzentrate zum Gegenstand.

In der **EP-A2 0353580** (Th.Goldschmidt) wird vorgeschlagen, konzentrierte, fließfähige wäßrige, gegebenenfalls niedere aliphatische Alkohole enthaltende Lösungen von Betainen herzustellen, in dem man die Quaternierung in wäßriger oder wäßrig-alkoholischer Lösung unter Zusatz einer solchen Menge nichtionischer Tenside durchführt, daß die resultierende Lösung einen Niotensidgehalt von vorzugsweise 3 bis 20 Gew.-% aufweisen. Als geeignete nichtionische Tenside kommen hierbei vor allem Fettsäurepolyethylenoxidester in Betracht. Aus der **DE-A1 4305083** (Henkel) ist ferner bekannt, daß man die Quaternierung von Aminen in Abwesenheit von Wasser und organischen Lösungsmitteln auch in Gegenwart von Fettalkoholpolyglycolethern durchführen kann.

Die WO-9 325 650 offenbart Konzentrate auf Basis von Alkylpolyglucosiden und anionischen bzw. amphoteren Tensiden, die als zwingende Komponente einen Zusatz anorganischer bzw. organischer Elektrolytsalze enthalten.

Im Markt besteht ein Bedürfnis nach konzentrierten Tensidmischungen auf Basis von Alkyl- und/oder Alkenyloligoglucosiden, die bei einem Feststoffgehalt oberhalb von 30 Gew.-% und vorzugsweise von etwa 40 bis 50 Gew.-% fließ- und pumpfähig sind sowie eine signifikant verminderte Kristallisationsneigung, d.h. eine verbesserte Lagerstabilität aufweisen. Da derartige Tensidcompounds überwiegend im Kosmetiksektor Verwendung finden, ist die hautkosmetische bzw. dermatologische Verträglichkeit ebenfalls überaus wichtig.

Die Tensidkonzentrate stellen für Hersteller und Anwender eine besonders vorteilhafte Handelsform dar, da sie hinsichtlich ihres Wassergehaltes minimiert worden sind und somit geringere Kosten bei Transport und Lagerung verursachen. Gleichwohl ist erwünscht, daß die Tensidkonzentrate bei Einsatz in den Endformulierungen, die naturgemäß stark verdünnt sind und einen Feststoffgehalt von 20 bis 30 Gew.-% aufweisen, eine ausreichend hohe Viskosität aufweisen bzw. sich problemlos unter Einsatz bekannter Zusatzstoffe verdicken lassen.

Die komplexe Aufgabe der Erfindung hat somit darin bestanden pumpfähige wäßrige Tensidkonzentrate mit guter hautkosmetischer Verträglichkeit auf Basis von Alkyl- und/oder Alkenyloligoglykosiden zur Verfügung zu stellen, die sich durch eine hohe Lagerstabilität auszeichnen, eine Viskosität nach Brookfield von maximal 10.000 mPas sowie einen Feststoffgehalt von 30 bis 50 Gew-% aufweisen und sich bei Einarbeitung in kosmetische Mittel mit einem Wassergehalt von mindestens 50 Gew.-% problemlos auf eine Viskosität von mindestens 2000 mPas verdicken lassen.

### Beschreibung der Erfindung

Gegenstand der Erfindung ist ein Verfahren zur Herstellung pumpfähiger, wäßriger Tensidkonzentrate, bei dem man sekundäre oder tertiäre Amine in Gegenwart von Alkylund/oder Alkenyloligoglykosiden in an sich bekannter Weise mit Alkylierungsmitteln umsetzt und im Anschluß an die Alkylierung eine Drucknachbehandlung bei Temperaturen im Bereich von 80 bis 140°C, Drücken im Bereich von 1 bis 10 bar und pH-Werten von 8 bis 13 durchführt. Hierzu ist anzumerken, daß die Herstellung der erfindungsgemäßen Tensidkonzentrate mit dem gewünschten Feststofigehalt auf anderem Wege, d.h. beispielsweise durch Vermischen der wäßrigen Einzelkomponenten oder Eintragen von pulverförmigen Alkylglucosiden in wäßrige Betainpasten nicht ohne weiteres möglich ist.

Überraschenderweise wurde gefunden, daß die so hergestellten Tensidkonzentrate eine ausgezeichnete hautkosmetische Verträglichkeit besitzen, gute Schaumeigenschaften auch in Abmischung mit weiteren Tensiden wie insbesondere Fettalkoholethersulfaten zeigen und über eine sehr gute Lagerstabilität auch bei niedrigen Temperaturen verfügen. Insbesondere die Ausbildung von Kristallen, wie sie von wäßrigen Alkylglucosidpasten bekannt ist, wird zuverlässig vermieden. Die Erfindung schließt ferner die Erkenntnis ein, daß die Tensidkonzentrate die erforderlich niedrige Viskosität von weniger als 10.000 und vorzugsweise 3.000 bis 7.500 mPas (bestimmt nach der Brookfield-Methode) aufweisen, sich jedoch in verdünnten wäßrigen Formulierungen problemlos auf eine Viskosität von mindestens 2.000 mPas verdicken lassen.

### Amine

Als Aminkomponenten kommen sekundäre, insbesondere tertiäre Amine in Betracht. Geeignete Ausgangsstoffe sind beispielsweise **Di-, vorzugsweise Trialkylamine** der Formel **(I)**, 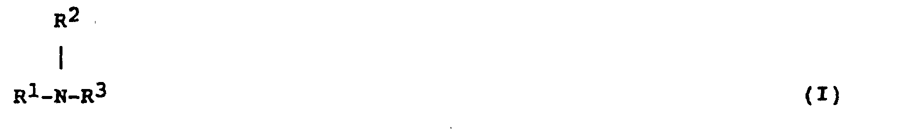 in der R¹ für Alkyl- und/oder Alkenylreste mit 6 bis 22 Kohlenstoffatomen, R² für Wasserstoff oder Alkylreste mit 1 bis 4 Kohlenstoffatomen und R³ für Alkylreste mit 1 bis 4 Kohlenstoffatomen steht.

Typische Beispiele sind Hexylmethylamin, Hexyldimethylamin, Octyldimethylamin, Decyldimethylamin, Dodecylmethylamin, Dodecyldimethylamin, Dodecylethylmethylamin, C_{12/14}-Kokosalkyldimethylamin, Myristyldimethylamin, Cetyldimethylamin, Stearyldimethylamin, Stearylethylmethylamin, Oleyldimethylamin, C_{16/18}-Talgalkyldimethylamin sowie deren technische Gemische.

Als weitere Einsatzstoffe kommen auch Amidoamine der Formel (II) in Betracht, 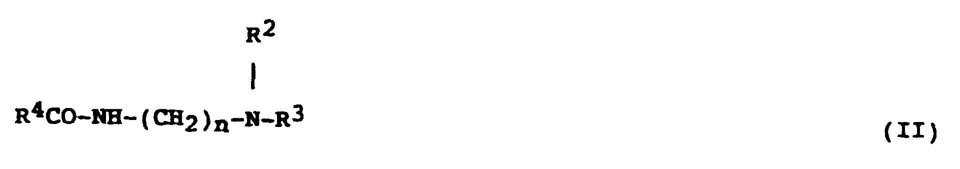 in der R⁴CO für einen aliphatischen Acylrest mit 6 bis 22 Kohlenstoffatomen und 0 oder 1 bis 3 Doppelbindungen, n für Zahlen von 1 bis 3 steht und R² und R³ die oben angegebenen Bedeutungen haben.

Amidoamine stellen bekannte Stoffe dar, die nach den einschlägigen Verfahren der präparativen Chemie erhalten werden können. Ein Verfahren zu ihrer Herstellung besteht beispielsweise darin, Fettsäuren mit Diaminen zu amidieren. Typische Beispiele sind Umsetzungsprodukte von Fettsäuren mit 6 bis 22 Kohlenstoffatomen, namentlich Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Gemische, mit N,N-Dimethylaminoethylamin, N, N-Dimethylaminopropylamin, N,N-Diethylaminoethylamin und N,N-Diethylaminopropylamin. Bevorzugt ist der Einsatz von C_{8/18}-Kokosfettsäure-N,N-dimethylaminopropylamid.

Weiterhin kommen als geeignete Amine Imidazoline der Formel (III) in Betracht, 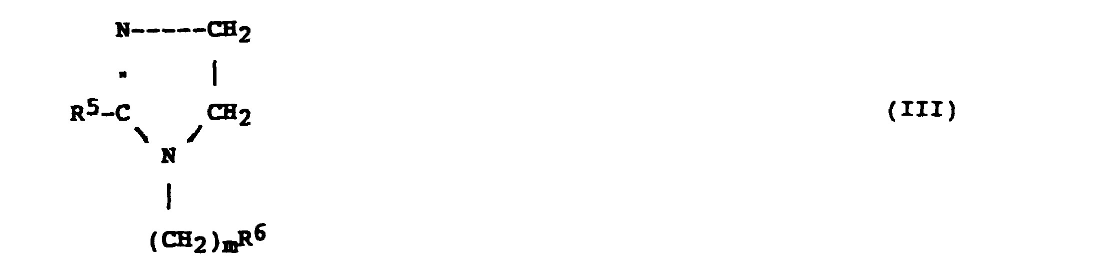 in der R⁵ für einen Alkylrest mit 5 bis 21 Kohlenstoffatomen, R⁶ für eine Hydroxylgruppe, einen OCOR⁵- oder NHCOR⁵-Rest und m für 2 oder 3 steht.

Auch bei diesen Substanzen handelt es sich um bekannte Stoffe, die beispielsweise durch cyclisierende Kondensation von 1 oder 2 Mol Fettsäure mit mehrwertigen Aminen wie beispielsweise Aminoethylethanolamin (AEEA) oder Diethylentriamin erhalten werden können.

Typische Beispiele sind Kondensationsprodukte der oben genannten Fettsäuren mit AEEA, vorzugsweise Imidazoline auf Basis von Laurinsäure oder wiederum C_{12/14}-Kokosfettsäure.

### Alkyl- und/oder Alkenyloligoglykoside

Alkyl- und/oder Alkenyloligoglykoside stellen bekannte Stoffe dar und folgen der Formel **(IV)**,

R⁷O-[G]ₚ (IV)

in der R⁷ für einen Alkyl- und/oder Alkenylrest mit 10 bis 22 Kohlenstoffatomen, G für einen Zuckerrest mit 5 oder 6 Kohlenstoffatomen und p für Zahlen von 1 bis 10 steht. Alkyl- und/oder Alkenyloligoglykoside können nach den einschlägigen Verfahren der präparativen organischen Chemie erhalten werden; stellvertretend für das umfangreiche Schrifttum sei hier auf die Schriften **EP-A1 0301298** und **WO 90103977** verwiesen.

Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/ oder Alkenyloligoglykoside sind somit Alkylund/oder Alkenyloligo**glucoside**.

Die Indexzahl p in der allgemeinen Formel **(IV)** gibt den Oligomerisierungsgrad (DP-Grad), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,5 liegt.

Der Alkyl- bzw. Alkenylrest R⁷ kann sich von primären Alkoholen mit 10 bis 22, vorzugsweise 12 bis 16 Kohlenstoffatomen ableiten. Typische Beispiele sind Caprylalkohol, Caprinalkohol, Undecylalkohol, Laurylalkohol, Myristylalkohol, Cetylalkohol, Palmoleylalkohol, Stearylalkohol, isoslaarylalkchol, Oleylalkohol, Elaidylalkohol, Petroselinylalkohol, Arachylalkohol, Gadoleylalkohol, Behenylalkohol, Erucylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese anfallen. Bevorzugt sind Alkyloligoglucoside auf Basis von gehärtetem C_{12/14}- Kokosalkohol mit einem DP von 1 bis 3.

Üblicherweise setzt man die Amine und die Glykoside im Gewichtsverhältnis 10 : 90 bis 90 : 10, vorzugsweise 20 : 80 bis 80 : 20 und insbesondere im Verhältnis 40 : 60 bis 60 : 40 ein.

### Alkylierungsmitte

Als Alkylierungsmittel können Halogencarbonsäuren bzw. deren Alkalisalze oder Ester eingesetzt werden. Aus Gründen der leichten Verfügbarkeit ist der Einsatz von Natriumchloracetat bevorzugt. Die Amine und die Halogencarbonsäuren bzw. deren Salze können im molaren Verhältnis von 1 : 1,0 bis 1 : 2,5, vorzugsweise 1 : 1 bis 1 : 2,0 eingesetzt werden. Ein hohes Einsatzverhältnis ist insbesondere dann vorteilhaft, wenn als Amine Imidazoline eingesetzt werden.

### Quaternierung

Die Quaternierung bzw. Betainisierung der Amine kann in an sich bekannter Weise durchgeführt werden. Es empfiehlt, sich die Reaktion in einer solchen Menge Wasser als Lösungsmittel durchzuführen, daß sich in den resultierenden Tensidkonzentraten ein Feststoffgehalt von 30 bis 50 und vorzugsweise 43 bis 48 Gew.-% einstellt. Anstelle von Wasser können auch Fettalkoholpolyglycolether, ethoxylierte Partialglyceride, Niedrigalkylglykoside mit 4 bis 8 Kohlenstoffatomen im Alkylrest und dergleichen eingesetzt werden. Es ist ferner möglich beliebige Mischungen von Wasser und den genannten Solventien als Lösungsmittel einzusetzen. Üblicherweise wird die Alkylierung bei Temperaturen im Bereich von 70 bis 98°C durchgeführt und ist innerhalb von 1 bis 10, vorzugsweise 2 bis 5 h beendet.

Im Hinblick auf eine möglichst vollständige Abreaktion des Alkylierungsmittels hat es sich als vorteilhaft erwiesen, die Reaktion bei einem pH-Wert von 6 bis 10, vorzugsweise 7 bis 8,5 zu führen. Eine weitere Möglichkeit zur Minimierung des Restgehaltes an Alkylierungsmittel besteht ferner darin, der Reaktionsmischung nach Beendigung der Alkylierung einen definierten Überschuß an Aminosäuren, insbesondere Glycin zuzusetzen, wie dies in der **DE-A1 3939264** (Henkel) beschrieben wird.

### Gewerbliche Anwendbarkeit

Die erfindungsgemäßen wäßrigen Tensidkonzentrate weisen einen Feststoffgehalt im Bereich von 30 bis 50 Gew.-% auf. Sie sind lagerstabil, neigen nicht zur Kristallisation, besitzen eine Viskosität von weniger als 10.000 mPas und sind dementsprechend pumpfähig. In verdünnten wäßrigen Formulierungen zeigen sie eine gute Wiederverdickbarkeit und eine ausgezeichnete hautkosmetische Verträglichkeit. Sofern die Tensidkonzentrate bereits ein Verdikkungsmittel enthalten, kann in vielen Fällen auf den weiteren Zusatz von Verdickungsmitteln, vorzugsweise Fettalkoholpolyglycolethern mit eingeengter Homologenverteilung, zu den kosmetischen Mitteln sogar verzichtet werden, da sich die gewünschte Viskosität automatisch einstellt. Ein weiterer Gegenstand der Erfindung betrifft daher die Verwendung der erfindungsgemäßen Tensidkonzentrate zur Herstellung von oberflächenaktiven Mitteln, wie z.B. Handgeschirrspülmitteln sowie insbesondere Mitteln zur Haut- und Haarpflege.

### Haut- und Haarpflegemittel

Die Haut- und Haarpflegemittel können in untergeordneten Mengen weitere, mit den anderen Inhaltsstoffen kompatible Tenside enthalten. Typische Beispiele sind Fettalkoholpolyglycolethersulfate, Monoglyceridsulfate, Ethercarbonsäuren, Fettsäureisethionate, Fettsäuresarcosinate, Fettsäuretauride, Acylglutamate und/oder Proteinhydrolysate bzw. deren Kondensate mit Fettsäuren auf tierischer oder vorzugsweise pflanzlicher Basis.

**Hautpflegemittel**, wie Cremes, Lotionen und dergleichen, weisen in der Regel - neben den bereits genannten Tensiden - einen Gehalt an Ölkörpern, Emulgatoren, Fetten und Wachsen, Stabilisatoren sowie ebenfalls Überfettungsmitteln, Verdikkungsmitteln, biogenen Wirkstoffen, Filmbildnern, Konservierungsmitteln, Farb- und Duftstoffen auf.

**Haarpflegemittel**, wie beispielsweise Haarshampoos, Haarlotionen, Schaumbäder und dergleichen, können als weitere Hilfs- und Zusatzstoffe - neben den bereits genannten Tensiden - Emulgatoren, Überfettungsmittel, Verdickungsmittel, biogene Wirkstoffe, Filmbildner, Konservierungsmittel, Farb- und Duftstoffe enthalten.

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₀-Fettsäuren mit linearen C₆- C₂₀-Fettalkoholert, Ester von verzweigten C₆-C₁₃-Carbonsäuren mit linearen C₁₆-C₁₈-Fettalkoholen, Ester von linearen C₁₀-C₁₈-Fettsäuren mit verzweigten Alkoholen, insbesondere 2-Ethylhexanol, Ester von linearen und/oder verzweigten Fettsäuren mit zweiwertigen Alkoholen und/oder Guerbetalkoholen, Triglyceride auf Basis C₆-C₁₀-Fettsäuren, pflanzliche Öle, verzweigte primäre Alkohole, substituierte Cyclohexane, Guerbetcarbonate und/oder Dialkylether in Betracht.

Als **Emulgatoren** kommen sowohl bekannte W/O- als auch O/W-Emulgatoren wie beispielsweise gehärtetes und ethoxyliertes Ricinusöl, Polyglycerinfettsäureester oder Polyglycerinpolyricinoleate in Frage.

Typische Beispiele für Fette sind Glyceride, als **Wachse** kommen u.a. Bienenwachs, Paraffinwachs oder Mikrowachse gegebenenfalls in Kombination mit hydrophilen Wachsen, z.B. Cetylstearylalkohol in Frage.

Als **Stabilisatoren** können Metallsalze von Fettsäuren wie z.B. Magnesium-, Aluminium und/oder Zinkstearat eingesetzt werden.

Als **Überfettungsmittel** können Substanzen wie beispielsweise polyethoxylierte Lanolinderivate, Lecithinderivate, Polyolfettsäureester, Monoglyceride und Fettsäurealkanolamide verwendet werden, wobei die letzteren gleichzeitig als Schaumstabilisatoren dienen.

Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, Polyvinylalkohol und Polyvinylpyrrolidon, Tenside wie beispielsweise Fettalkoholethoxylate mit eingeengter Homologenverteilung oder Alkyloligoglucoside sowie Elektrolyte wie Kochsalz und Ammoniumchlorid.

Unter **biogenen Wirkstoffen** sind beispielsweise Pflanzenextrakte und Vitaminkomplexe zu verstehen.

Gebräuchliche **Filmbildner** sind beispielsweise Chitosan, mikrokristallines Chitosan, quaterniertes Chitosan, Polyvinylpyrrolidon, Vinylpyrrolidon-Vinyl-acetat-Copolymerisate, Polymere der Acrylsäurereihe, quaternäre Cellulose-Derivate und ähnliche Verbindungen.

Als **Konservierungsmittel** eignen sich beispielsweise Phenoxyethanol, Formaldehydlösung, Parabene, Pentandiol oder Sorbinsäure.

Als **Perlglanzmittel** kommen beispielsweise Glycoldistearinsäureester wie Ethylenglycoldistearat, aber auch Fettsäuremonoglycolester in Betracht.

Als Farbstoffe können die für kosmetische Zwecke geeigneten und zugelassenen Substanzen verwendet werden, wie sie beispielsweise in der Publikation "**Kosmetische Färbemittel" der Farbstoffkommission der** Deut**schen Forschungsgemeinschaft, veröffentlicht im Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% und der nicht wäßrige Anteil ("Aktivsubstanzgehalt") 20 bis 80, vorzugsweise 30 bis 70 Gew.-% - bezogen auf die Mittel - betragen. Die Herstellung der Mittel kann in an sich bekannter Weise, d.h. beispielsweise durch Heiß-, Kalt-, Heiß-Heiß/Kalt- bzw. PIT-Emulgierung erfolgen. Hierbei handelt es sich um ein rein mechanisches Verfahren, eine chemische Reaktion findet nicht statt.

Die folgenden Beispiele sollen den Gegenstand der Erfindung näher erläutern, ohne ihn darauf einzuschränken.

### Beispiele

### I. Herstellbeispiele

### Beispiel H1:

### C_{8/18}-Kokosfettsäureamidopropyl-N,N-dimethylaminobetain/C_{12/14}-Alkyloligoglucosid ("Compound A").

In einem 2-I-Vierhalskolben, ausgerüstet mit Rührer, Thermometer, Tropftrichter und pH-Elektrode, wurden 44,6 g (0,375 Mol) Natriumchloracetat (96 Gew.-%ig) zusammen mit 145,3 g Wasser und 1204,2 g C_{12/14}-Kokosalkyloligoglucosid (Plantaren^{(R)} APG 600 CS UP, Henkel KGaA, Düsseldorf(FRG); 50 Gew.-%ige wäßrige Paste) bei 40°C homogenisiert. Danach wurden 102,7 g (0,33 Mol) gehärtetes C_{8/18}-Kokosfettsäureamidopropyl-N,N-dimethylamin zugegeben und die Mischung bei 90°C gerhalten. Der Initial-pH-Wert betrug aufgrund der alkalischen Einstellung des Glykosids ca. 11 und fiel im Laufe der 3-stündigen Umsetzung auf einen Wert von 9,7 ab. Zu diesem Zeitpunkt war das Amidoamin vollständig abreagiert. Es wurde eine pumpfähige Flüssigkeit der folgenden Zusammensetzung erhalten:

| | |
|---|---|
| 7,7 Gew.-% | Betain |
| 40,2 Gew.-% | Alkyloligolucosid |
| 1,3 Gew.-% | Natriumchlorid |
| 50,4 Gew.-% | Wasser |
| 0,1 Gew.-% | Natriumchloracetat |

Das Rohprodukt wurde in einen Autoklaven überführt und mit einer solchen Menge 50 Gew.-%iger Natriumhydroxidlösung versetzt, daß bezogen auf eine 10 Gew.-%ige Produktverdünnung ein pH-Wert von 11,5 resultierte. Anschließend wurde die Mischung über einen Zeitraum von 60 min bei einer Temperatur von 120°C und einem autogenen Druck von 2 bar nachbehandelt, wobei der Restgehalt an Natriumchloracetat auf einen Wert < 5 ppm minimiert wurde.

### Beispiel H1:

### C_{12/18}-Kokosalkyl-H,H-dimethylaminobetain/C_{12/14}-Alkyloligoglucosid ("Compound B").

Analog Beispiel H1 wurden 85,2 g (0,72 Mol) Natriumchloracetat, 235,0 g Wasser und 1050 g C_{12/14}-Kokosoligoglucosid mit 139,7 g (0,63 Mol) Kokosdimethylamin gemischt und bei 90°C zur Reaktion gebracht. Der Initial-pH-Wert betrug 10,4 und sank im Laufe einer Reaktionszeit von 2,5 h auf 8,5 ab; der Restamingehalt betrug zu diesem Zeitpunkt 0,3 Gew.-%. Es wurde eine gut fließfähige Flüssigkeit der folgenden Zusammensetzung erhalten:

| | |
|---|---|
| 11,7 Gew.-% | Betain |
| 35,0 Gew.-% | Alkyloligoglucosid |
| 2,8 Gew.-% | Natriumchlorid |
| 48,3 Gew.-% | Wasser |
| 0,3 Gew.-% | Natriumchloracetat |

Der Anteil an Natriumchloracetat wurde durch eine Nachbehandlung analog Beispiel H 1 auf 9 ppm abgesenkt.

### II. Untersuchung der Konzentrate

Die Viskosität der folgenden Gemische wurde nach der Brookfield-Methode (23°C, Spindel 2, 200 Upm) bestimmt. Die Stabilität der Gemische wurde nach Lagerung über 4 Wochen bei 5°C visuell beurteilt. Dabei bedeuten:

| | |
|---|---|
| +++ | = klare Lösung, keine Kristallbildung |
| ++ | = vereinzelte Kristallbildung |
| + | = deutliche Kristallbildung |
| - | = Teile der Lösung durchkristallisiert |
| * | = trübes Produkt, instabil, Phasentrennung |

Die Zusammensetzungen sowie die Daten zur Viskosität und zur Stabilität sind in den Tabellen 1 und 2 zusammengefaßt. Die Angaben zu den Zusammensetzungen der Gemische (in Gew.-%) verstehen sich bezogen auf den Feststoffgehalt der Komponenten. Alle Gemische wurden mit einem Wassergehalt von 55 Gew.-% (pH-Wert = 5,3) eingesetzt.

Eine Erläuterung der Handelsnamen befindet sich in Tabelle 4.

**Tabellen 1 und 2**

| Erfindungsgemäße Tensidkonzentrate und Vergleichsmischungen | | | | | |
|---|---|---|---|---|---|
| **Komponenten** | **1** | **2** | **3** | **4** | **5** |
| Compound A | 96,0 | - | 97,0 | 97,5 | - |
| Compound B | - | 97,0 | - | - | 96,0 |
| Arlypon^{(R)} F | 1,0 | 1,0 | 0,5 | - | 1,5 |
| Konservierungsmt. | 0,1 | 0,1 | 0,1 | 0,1 | 0,1 |
| Milchsäure 85%ig | 2,9 | 1,9 | 2,4 | 2,4 | 2,4 |
| Viskosität [mPas] | 7000 | 4900 | 4250 | 3700 | 4900 |
| Stabilität | +++ | +++ | +++ | +++ | +++ |

| **Komponenten** | **V1** | **V2** | **V3** | **V4** | **V5** |
|---|---|---|---|---|---|
| Plantaren^{(R)} 600 | 50,0 | - | - | 50,0 | 50,0 |
| Plantaren^{(R)} 2000 | - | 50,0 | - | - | - |
| Texapon^{(R)} SB 3 | 46,0 | 46,0 | 50,0 | 40,0 | 6,0 |
| Dehyton^{(R)} K | - | - | 46,0 | 6,0 | 40,0 |
| Arlypon^{(R)} F | 1,0 | 1,0 | 1,0 | 1,0 | 1,0 |
| Konservierungsmt. | 0,1 | 0,1 | 0,1 | 0,1 | 0,1 |
| Milchsäure 85%ig | 2,9 | 2,9 | 2,9 | 2,9 | 2,9 |
| Viskosität [mPas] | 14000 | 12000 | 5600 | 9400 | 9400 |
| Stabilität | - | - | * | ++ | + |

### III. Untersuchung der verdünnten Anwendungsformulierungen

Die erfindungsgemäßen Konzentrate 1 bis 5 sowie die Vergleichsmischungen V1 bis V5 wurden in die Rezeptur einer verdünnten kosmetischen Hautlotion eingesetzt:

| | |
|---|---|
| Tensidkonzentrat (berechnet wasserfrei) | 10,0 Gew.-% |
| Dehyton^{(R)} G | 5,0 Gew.-% |
| Lamepon^{(R)} S | 2,5 Gew.-% |
| Euperlan^{(R)} PK 900 | 1,5 Gew.-% |
| Cetiol^{(R)} HE | 1,0 Gew.-% |
| Lamesoft^{(R)} LMG | 2,5 Gew.-% |
| Farbstoff | 1,0 Gew.-% |
| Konservierungsmittel | 0,1 Gew.-% |
| Wasser | 76,4 Gew.-% |

Die Ergebnisse sind in Tabelle 3 zusammengefaßt:

**Tabelle 3**

| Viskosität in der Anwendungskonzentration | | | |
|---|---|---|---|
| **Bsp.** | **pH-Wert** | **Viskosität mPas** | **Viskosität bei Zugabe 1 Gew.-% Arlypon**^{**(R)**} **F mPas** |
| 1 | 5,3 | 4400 | |
| 2 | 5,2 | 4100 | |
| 3 | 5,3 | 4300 | |
| 4 | 5,4 | 4350 | |
| 5 | 5,3 | 4300 | |
| V1 | 5,3 | 980 | 1200 |
| V2 | 5,3 | 940 | 1100 |
| V3 | 5,3 | 1050 | 1200 |
| V4 | 5,3 | 990 | 1100 |
| V5 | 5,4 | 960 | 1100 |

**Tabelle 4**

| Erläuterung der Handelsnamen | |
|---|---|
| **Handelsnamen** | **CTFA-Registrierung** |
| Arlypon^{(R)} F | Laureth-2 |
| Cetiol^{(R)} HE | PEG-7 Glyceryl Cocoate |
| Dehyton^{(R)} G | Cocoamphodiacetate |
| Dehyton^{(R)} K | Cocamidopropyl Betaine |
| Euperlan^{(R)} PK 900 | PEG-3 Distearate (and) Sodium Laureth Sulfate |
| Lamepon^{(R)} S | Potassium-Cocoyl Hydrolyzed Collagen |
| Lamesoft^{(R)} LMG | Glyceryl Laurate (and) Potassium-Cocoyl Hydrolyzed Collagen |
| Plantaren^{(R)} 600 | Lauryl Polyglucose |
| Plantaren^{(R)} 2000 | Decyl Polyglucose |
| Texapon^{(R)} SB3 | Disodium Laurethsulfosuccinat |

## Patentansprüche

1. Verfahren zur Herstellung pumpfähiger, wäßriger Tensidkonzentrate, **dadurch gekennzeichnet, daß** man sekundäre oder tertiäre Amine in Gegenwart von Alkylund/oder Alkenyloligoglykosiden in an sich bekannter Weise mit Alkylierungsmitteln umsetzt und im Anschluß an die Alkylierung eine Drucknachbehandlung bei Temperaturen im Bereich von 80 bis 140°C, Drücken im Bereich von 1 bis 10 bar und pH-Werten von 8 bis 13 durchführt.

2. Verfahren nach Anspruch 1, **dadurch gekennzeichnet, daß** man sekundäre oder tertiäre Amine der Formel **(I)** einsetzt, 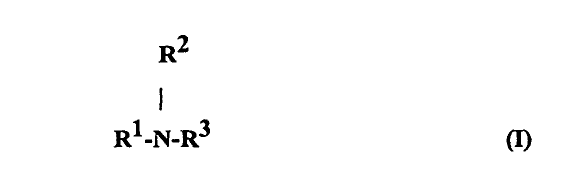 in der R¹ für Alkyl- und/oder Alkenylreste mit 6 bis 22 Kohlenstoffatomen, R² für Wasserstoff oder Alkylreste mit 1 bis 4 Kohlenstoffatomen und R³ für Alkylreste mit 1 bis 4 Kohlenstoffatomen steht.

3. Verfahren nach Anspruch 1, **dadurch gekennzeichnet, daß** man Amidoamine der Formel **(II)** einsetzt, 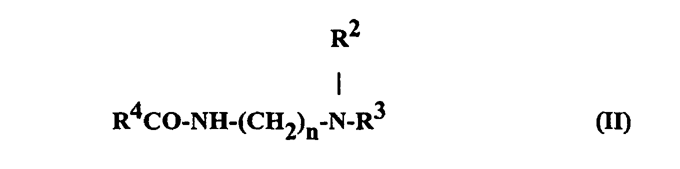 in der R⁴CO für einen aliphatischen Acylrest mit 6 bis 22 Kohlenstoffatomen und 0 oder 1 bis 3 Doppelbindungen, n für 0 oder Zahlen von 1 bis 3 steht und R² und R³ die oben angegebenen Bedeutungen haben.

4. Verfahren nach Anspruch 1, **dadurch gekennzeichnet, daß** man Imidazoline der Formel **(III)** einsetzt, 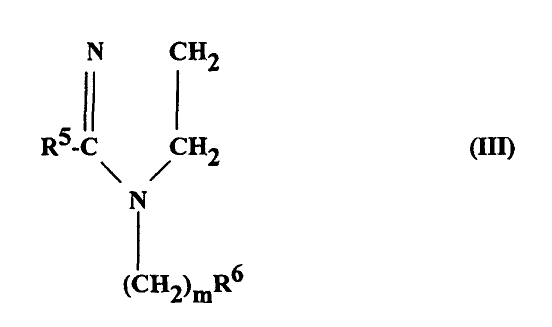 in der R⁵ für einen Alkylrest mit 5 bis 21 Kohlenstoffatomen, R⁶ für eine Hydroxylgruppe, einen OCOR⁵- oder NHCOR⁵-Rest und m für 2 oder 3 steht.

5. Verfahren nach den Ansprüchen 1 bis 4, **dadurch gekennzeichnet, daß** man Alkylund/oder Alkenyloligoglykoside der Formel **(IV)** einsetzt,
**R**^{**7**}**O-[G]**_{**p**} **(IV)**
in der R⁷ für einen Alkyl- und/oder Alkenylrest mit 10 bis 22 Kohlenstoffatomen, G für einen Zuckerrest mit 5 oder 6 Kohlenstoffatomen und p für Zahlen von 1 bis 10 steht.

6. Verfahren nach den Ansprüchen 1 bis 5, **dadurch gekennzeichnet, daß** man die Amine und die Glykoside im Gewichtsverhältnis 20 : 80 bis 80 : 20 einsetzt.

7. Verfahren nach den Ansprüchen 1 bis 6, **dadurch gekennzeichnet, daß** man als Alkylierungsmittel Halogencarbonsäuren bzw. deren Alkalisalze oder Ester einsetzt.

8. Verfahren nach den Ansprüchen 1 bis 7, **dadurch gekennzeichnet, daß** man die Amine und die Halogencarbonsäuren bzw. deren Salze im molaren Verhältnis von 1 : 1,0 bis 1 : 1,2 einsetzt.

9. Verfahren nach den Ansprüchen 1 bis 8, **dadurch gekennzeichnet, daß** man Wasser als Lösungsmittel in einer solchen Menge einsetzt, daß sich in den resultierenden Tensidkonzentraten ein Feststoffgehalt von 30 bis 50 Gew.-% einstellt.

10. Verfahren nach den Ansprüchen 1 bis 9, **dadurch gekennzeichnet, daß** man die Alkylierung bei Temperaturen im Bereich von 70 bis 98°C durchführt.

11. Verfahren nach den Ansprüchen 1 bis 10, **dadurch gekennzeichnet, daß** man die Alkylierung über einen Zeitraum von 1 bis 10 h durchführt.

12. Verfahren nach den Ansprüchen 1 bis 11, **dadurch gekennzeichnet, daß** man die Alkylierung bei einem pH-Wert von 6 bis 10 durchführt.

## Claims

1. A process for the production of pumpable water-containing surfactant concentrates, **characterized in that** secondary or tertiary amines are reacted with alkylating agents in known manner in the presence of alkyl and/or alkenyl oligoglycosides and the alkylation step is followed by a pressure aftertreatment at temperatures of 80 to 140°C, pressures of 1 to 10 bar and pH values of 8 to 13.

2. A process as claimed in claim 1, **characterized in that** secondary or tertiary amines corresponding to formula **(I)**: 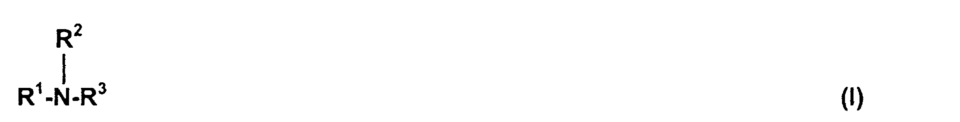 in which R¹ represents alkyl and/or alkenyl groups containing 6 to 22 carbon atoms, R² represents hydrogen or alkyl groups containing 1 to 4 carbon atoms and R³ represents alkyl groups containing 1 to 4 carbon atoms,
are used.

3. A process as claimed in claim 1, **characterized in that** amidoamines corresponding to formula **(II):** 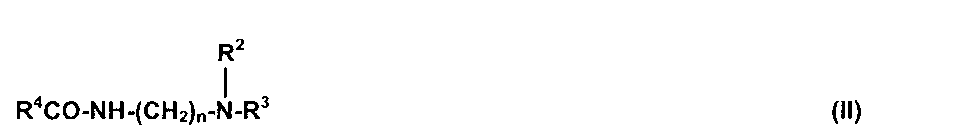 in which R⁴CO is an aliphatic acyl group containing 6 to 22 carbon atoms and 0 or 1 to 3 double bonds, n is 0 or a number of 1 to 3 and R² and R³ are as defined above,
are used.

4. A process as claimed in claim 1, **characterized in that** imidazolines corresponding to formula **(III):** 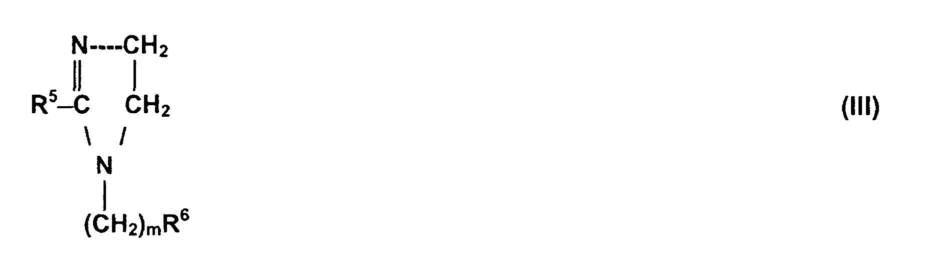 in which R⁵ is an alkyl group containing 5 to 21 carbon atoms, R⁶ is a hydroxyl group, an OCOR⁵ or NHCOR⁵ group and m = 2 or 3,
are used.

5. A process as claimed in claims 1 to 4, **characterized in that** alkyl and/or alkenyl oligoglycosides corresponding to formula **(IV):**
**R**^{**7**}**O-[G]**_{**P**} **(IV)**
in which R⁷ is an alkyl and/or alkenyl group containing 10 to 22 carbon atoms, G is a sugar unit containing 5 or 6 carbon atoms and p is a number of 1 to 10,
are used.

6. A process as claimed in claims 1 to 5, **characterized in that** the amines and the glycosides are used in a ratio by weight of 20:80 to 80:20.

7. A process as claimed in claims 1 to 6, **characterized in that** halocarboxylic acids or alkali metal salts or esters thereof are used as the alkylating agent.

8. A process as claimed in claims 1 to 7, **characterized in that** the amines and the halocarboxylic acids or their salts are used in a molar ratio of 1:1.0 to 1:1.2.

9. A process as claimed in claims 1 to 8, **characterized in that** water is used as solvent in such a quantity that the resulting surfactant concentrates have a solids content of 30 to 50% by weight.

10. A process as claimed in claims 1 to 9, **characterized in that** the alkylation is carried out at temperatures in the range from 70 to 98°C.

11. A process as claimed in claims 1 to 10, **characterized in that** the alkylation is carried out over a period of 1 to 10 h.

12. A process as claimed in claims 1 to 11, **characterized in that** the alkylation is carried out at a pH value of 6 to 10.

## Revendications

1. Procédé de fabrication de concentrés d'agents tensioactifs aqueux, pompables ,
**caractérisé en ce qu'**
on fait réagir des amines secondaires ou tertiaires en présence d'alkyl- et/ou alkényloligoglycosides, avec des agents d'alkylation d'une manière connue en soi, et on effectue en connexion à l'alkylation, un retraitement sous pression, à des températures dans la plage de 80 à 140°C, à des pressions dans la plage de 1 à 10 bars et à des valeurs de pH allant de 8 à 13.

2. Procédé selon la revendication 1,
**caractérisé en ce qu'**
on met en oeuvre des amines secondaires ou tertiaires de formule (I) : 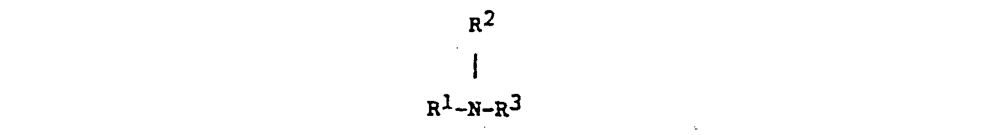 dans laquelle R¹ représente des radicaux alkyle et/ou alkényle ayant de 6 à 22 atomes de carbone,
R² représente de l'hydrogène ou des radicaux alkyle ayant de 1 à 4 atomes de carbone et
R³ représente des radicaux alkyle ayant de 1 à 4 atomes de carbone.

3. Procédé selon la revendication 1,
**caractérisé en ce qu'**
on met en oeuvre des amidoamines de formule (II) : 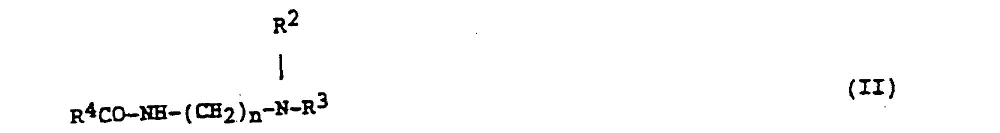 dans laquelle R⁴CO représente un radical acyle aliphatique ayant de 6 à 22 atomes de carbone et ayant 0 ou de 1 à 3 double liaisons,
n représente 0 ou des nombres allant de 1 à 3 et
R² et R³ ont les significations indiquées ci-dessus.

4. Procédé selon la revendication 1,
**caractérisé en ce qu'**
on met en oeuvre des imidazolines de formule (III) : 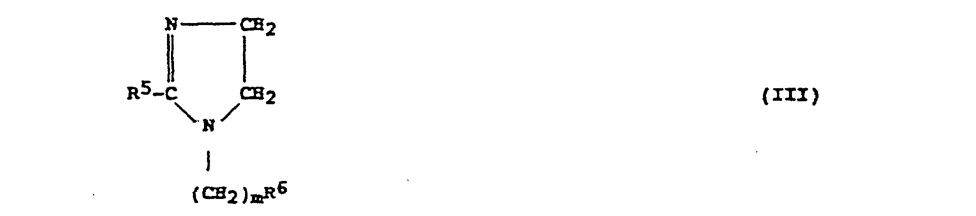 dans laquelle R⁵ représente un radical alkyle ayant de 5 à 21 atomes de carbone,
R⁶ représente un groupe hydroxyle, un reste OCOR⁵ ou NHCOR⁵ et
m représente 2 ou 3.

5. Procédé selon les revendications 1 à 4,
**caractérisé en ce qu'**
on met en oeuvre des alkyl- et/ou alkényloligoglycosides de formule (IV) :
R⁷O-[ G]p
dans laquelle R⁷ représente un radical alkyle et/ou alkényle ayant de 10 à 22 atomes de carbone,
G représente un radical de sucre ayant 5 ou 6 atomes de carbone et
p représente des nombres allant de 1 à 10.

6. Procédé selon les revendications 1 à 5,
**caractérisé en ce qu'**
on met en oeuvre les amines et les glycosides dans un rapport en poids allant de 20 :80 à 80 :20.

7. Procédé selon les revendications 1 à 6,
**caractérisé en ce qu'**
on met en oeuvre comme agent d'alkylation, des acides halogénocarboxyliques ou leurs sels de métal alcalin ou leurs esters.

8. Procédé selon les revendications 1 à 7,
**caractérisé en ce qu'**
on met en oeuvre les amines et les acides halogénocarboxyliques ou leurs sels, dans un rapport molaire allant de 1 :1,0 à 1 :1,2.

9. Procédé selon les revendications 1 à 8,
**caractérisé en ce qu'**
on met en oeuvre de l'eau comme solvant en une quantité telle que dans les concentrés d'agent tensioactif résultants, il s'ajuste une teneur en matière solide allant de 30 à 50 % en poids.

10. Procédé selon les revendications 1 à 9,
**caractérisé en ce qu'**
on effectue l'alkylation à des températures dans la plage de 70 à 98°C.

11. Procédé selon les revendications 1 à 10,
**caractérisé en ce qu'**
on effectue l'alkylation pendant un laps de temps allant de 1 à 10 heures.

12. Procédé selon les revendications 1 à 11,
**caractérisé en ce qu'**
on effectue l'alkylation à une valeur de pH de 6 à 10.
